# EUROPEAN PATENT APPLICATION

(11) **EP 3 097 880 A1**
(43) Date of publication of application: **30.11.2016**
(21) Application number: 15740270.2
(22) Date of filing: 22.01.2015
(51) Int. Cl.: A61B 18/12

(54) **THERAPEUTIC APPARATUS**

(30) Priority: 24.01.2014 JP 2014011843
(71) Applicant: Olympus Corporation, Tokyo 192-8507 (JP)
(72) Inventor: KUDO, Koichi, Hachioji-shi, Tokyo 192-8507 (JP)
(74) Representative: Gunzelmann, Rainer
(86) International application number: PCT/JP2015/051700
(87) International publication number: WO 2015/111662

(57) **Abstract**

A treatment apparatus (300) has a high frequency electrode (110). An electrothermal conversion element (140) with an electric resistance pattern (144) is adhered on the high frequency electrode (110). The electric resistance pattern (144) converts power to heat, and heats the high frequency electrode (110). Therefore, a body tissue in contact with the high frequency electrode (110) is cauterized. A current line (162) is connected to the high frequency electrode (110). The current line (162) is thick, and high in thermal conductivity, and the heat transferred from the electric resistance pattern (144) to the high frequency electrode (110) is thus transferred to the current line (162). For this reason, the temperature of the high frequency electrode (110) is less likely to increase near the current line (162). For compensation therefor, the electric resistance pattern (144) is densely formed near the current line (162). Therefore, the electric resistance pattern (144) generates heat at a high density near the current line (162), resulting in that the temperature of the high frequency electrode (110) increases in a relatively uniform manner.

## Description

### Technical Field

The present invention relates to a treatment apparatus.

### Background Art

There is generally known a treatment apparatus for treating body tissues by use of thermal energy. For example, Jpn. Pat. Appln. KOKAI Publication No. 2013-034568 discloses therein a treatment apparatus described later. More specifically, the treatment apparatus has an openable and closable high frequency electrode which grips a body tissue to be treated. A high frequency electrode current line for applying a high frequency voltage is connected to the high frequency electrode. In addition, the high frequency electrode also functions as a heat transfer plate which conducts heat to the gripped body tissue. The high frequency electrode has a sheet heater arranged as an electrothermal conversion element for heating the high frequency electrode. Accordingly, the treatment apparatus can apply a high frequency voltage to the gripped body tissue, and apply thermal energy on the body tissue. The treatment apparatus can cauterize and treat the body tissue with high frequency energy and thermal energy.

### Summary of Invention

The high frequency electrode current line is joined directly with the high frequency electrode, and excellent in thermal conductivity. For this reason, heat release from the high frequency electrode current line is significant in the case of the high frequency electrode which functions as a heat transfer plate. Consequently, the temperature of the high frequency electrode is likely to be non-uniform in the region with the high frequency electrode current line connected. However, the temperature of the high frequency electrode is preferably uniform.

It is an object of the present invention to provide a treatment apparatus which has a smaller temperature difference in a heat transfer plate.

To achieve the above described object, according to an aspect of the invention, a treatment apparatus configured to treat a body tissue includes a conductive heat transfer plate configured to transfer heat and power to the body tissue in contact with the body tissue, an electrothermal conversion element provided on the heat transfer plate, the electrothermal conversion element comprising an electric resistance pattern that generates heat with a voltage applied, and a lead wire configured to supply an electric current to the heat transfer plate, wherein heat generation density by the electric resistance pattern in a first region of a pattern region where the electric resistance pattern of the electrothermal conversion element is formed, the first region comprising a region where a distance from the lead wire is smaller than a predetermined value, is higher than heat generation density by the electric resistance pattern in a second region comprising at least a part of the pattern region other than the first region.

According to the present invention, the heat generation density of the electrothermal conversion element is adjusted for each region, and a treatment apparatus is thus provided which has a smaller temperature difference in a heat transfer plate.

### Brief Description of Drawings

FIG. 1 is a schematic diagram illustrating an exemplary structure of a treatment system according to each exemplary embodiment.
FIG. 2A is a schematic cross-section view illustrating an exemplary structure of a shaft and a holding part in an energy treatment tool according to each exemplary embodiment, which illustrates a state in which the holding part is closed.
FIG. 2B is a schematic cross-section view illustrating an exemplary structure of the shaft and the holding part in the energy treatment tool according to each exemplary embodiment, which illustrates a state in which the holding part is opened.
FIG. 3A is a plan view illustrating an exemplary structure of a first holding member in the holding part according to each exemplary embodiment.
FIG. 3B is a schematic diagram illustrating an exemplary structure of the first holding member in the holding part according to each exemplary embodiment, which is a longitudinal cross-section view along the line 3B-3B illustrated in FIG. 3A.
FIG. 3C is a schematic diagram illustrating an exemplary structure of the first holding member in the holding part according to each exemplary embodiment, which is a transverse cross-section view along the line 3C-3C illustrated in FIG. 3A.
FIG. 4 is an exploded perspective view illustrating an exemplary structure of a first electrode part according to a first exemplary embodiment.
FIG. 5 is a perspective view illustrating an exemplary structure of a first electrode part according to the first exemplary embodiment.
FIG. 6 is a perspective view illustrating an exemplary structure of a first high frequency electrode, a highly heat-conductive adhesive sheet, an electric resistance pattern, and a first high frequency electrode current line according to the first exemplary embodiment.
FIG. 7 is a perspective view illustrating an exemplary structure of a first high frequency electrode, a highly heat-conductive adhesive sheet, an electric resistance pattern, and a first high frequency electrode current line according to the second exemplary embodiment.

### Description of Embodiments

### First Exemplary Embodiment

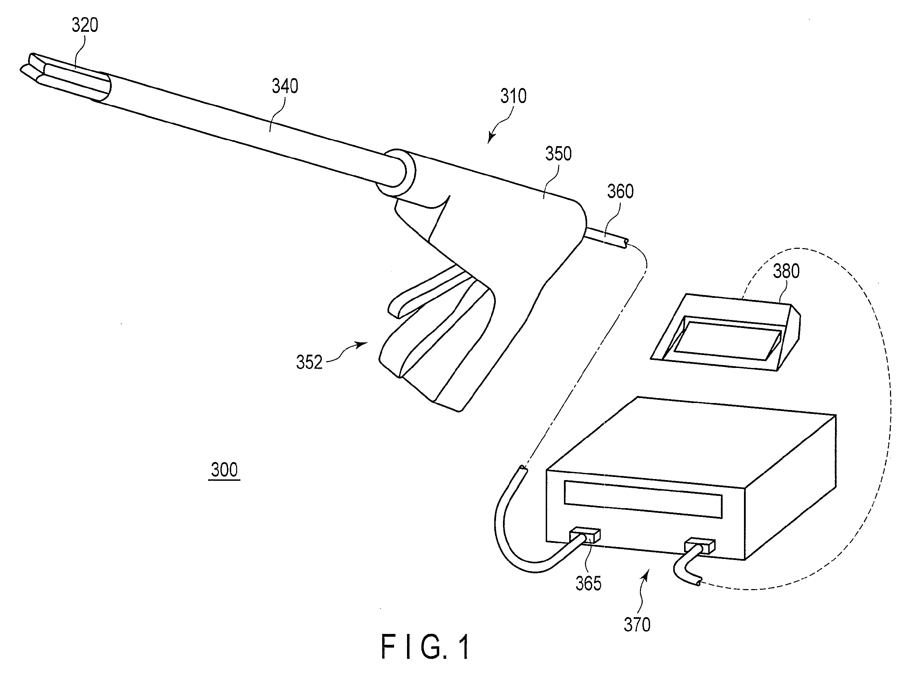

A first exemplary embodiment according to the present invention will be described with reference to the drawings. A treatment apparatus according to the present exemplary embodiment is used for treating body tissues. The treatment apparatus applies at least one of high frequency energy and thermal energy on body tissues. A treatment apparatus 300 is schematically illustrated in FIG. 1. As illustrated, the treatment apparatus 300 comprises an energy treatment tool 310, a control device 370, and a foot switch 380.

The energy treatment tool 310 is a linear type surgical treatment tool penetrating through the abdominal wall for treatment, for example. The energy treatment tool 310 includes a handle 350, a shaft 340 attached on the handle 350, and a holding part 320 provided on the tip end of the shaft 340. The holding part 320 is a treatment part which is openable and/or closable and is directed to perform treatments such as coagulation and incision of a body tissue by gripping the body tissue to be treated. For the following description, the side of the holding part 320 will be called tip end side and the side of the handle 350 will be called base end side. The handle 350 comprises a plurality of operation knobs 352 for operating the holding part 320.

A shape of the energy treatment tool 310 illustrated herein is exemplary, and any other shape having the same function may be employed. For example, the holding part, the handle, and the operation knobs may have different shapes from the shapes shown in FIG. 1, and the shaft may be curved. Furthermore, the energy treatment tool 310 is not limited to a forceps-like shape for gripping body tissues, but may be a trowel-type treatment tool which is pressed against body tissues in use.

The handle 350 is connected to the control device 370 via a cable 360. Herein, the cable 360 and the control device 370 are connected with each other via a connector 365, and the connection is removable to permit replacement of the energy treatment tool 310 in accordance with a treatment to be performed. The control device 370 is connected with the foot switch 380. The foot-operated foot switch 380 may be replaced with a hand-operated switch or another switch. An operator operates the pedal of the foot switch 380 thereby to switch ON/OFF energy supply from the control device 370 to the energy treatment tool 310.

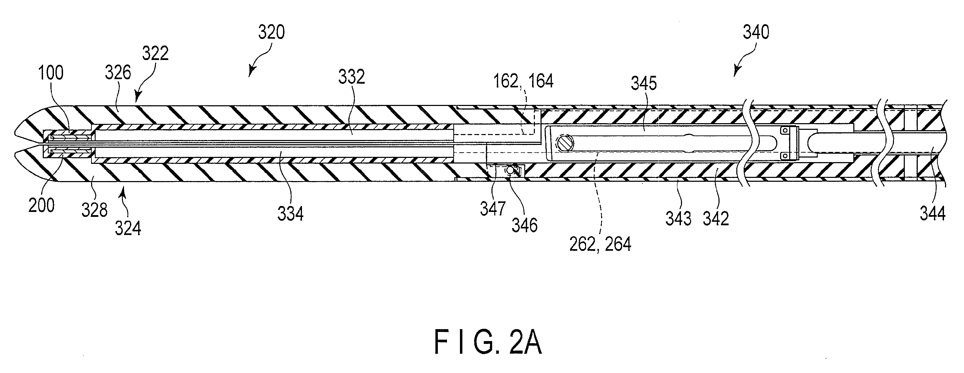

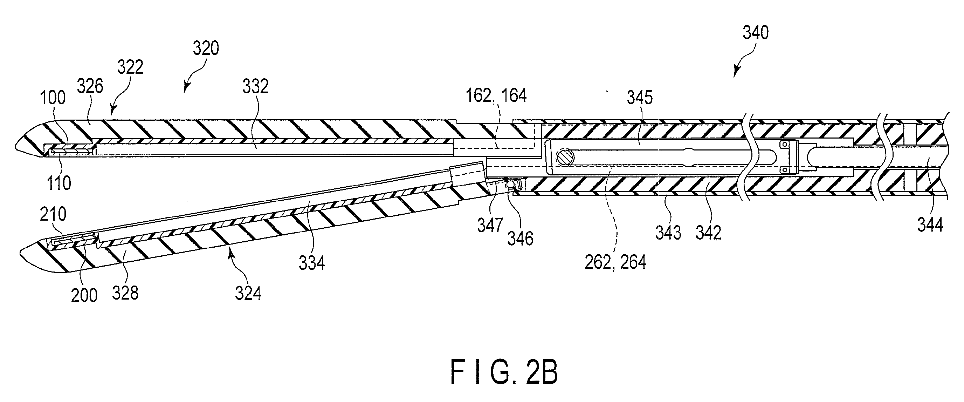

An exemplary structure of the holding part 320 and the shaft 340 is illustrated in FIG. 2A and FIG. 2B. FIG. 2A illustrates a state in which the holding part 320 is closed and FIG. 2B illustrates a state in which the holding part 320 is opened. The shaft 340 comprises a tube 342 and a sheath 343. The tube 342 is fixed at its base end to the handle 350. The sheath 343 is slidably arranged on the outer periphery of the tube 342 in the axial direction of the tube 342.

The holding part 320 is arranged on the tip end of the tube 342. The holding part 320 comprises a first holding member 322 and a second holding member 324. The base of the first holding member 322 is fixed on the tip end of the tube 342 in the shaft 340. On the other hand, the base of the second holding member 324 is rotatably supported on the tip end of the tube 342 in the shaft 340 by a support pin 346. Therefore, the second holding member 324 axially rotates about the support pin 346 and opens/closes relative to the first holding member 322.

In a state in which the holding part 320 is closed, a cross-section shape in which the base of the first holding member 322 and the base of the second holding member 324 are put together is circular. The second holding member 324 is energized by an elastic member 347 such as plate spring to open relative to the first holding member 322. When the sheath 343 is slid toward the tip end of the tube 342 so that the base of the first holding member 322 and the base of the second holding member 324 are covered by the sheath 343, as illustrated in FIG. 2A, the first holding member 322 and the second holding member 324 are closed against an energizing force of the elastic member 347. On the other hand, when the sheath 343 is slid toward the base end side of the tube 342, as illustrated in FIG. 2B, the second holding member 324 is opened relative to the first holding member 322 due to an energizing force of the elastic member 347.

The tube 342 is provided therein with a first high frequency electrode current line 162 connected to a first high frequency electrode 110 and a second high frequency electrode current line 262 connected to a second high frequency electrode 210, which will be described later. In addition, the tube 342 is provided therein with a pair of first heater current lines 164 connected to an electrothermal conversion element 140 to function as a heat generation member, which is arranged on the first high frequency electrode 110, and a pair of second heater current lines 264 connected to an electrothermal conversion element arranged on a second high frequency electrode 210.

A drive rod 344 connected on its base end to one of the operation knobs 352 is movably arranged in the axial direction of the tube 342 inside the tube 342. A thin plate-shaped cutter 345 forming a blade on its tip end is arranged on the tip end of the drive rod 344. When the operation knob 352 is operated, the cutter 345 is moved in the axial direction of the tube 342 via the drive rod 344. When the cutter 345 is moved toward the tip end, the cutter 345 is housed in a first cutter guide groove 332 and a second cutter guide groove 334 described later formed in the holding part 320.

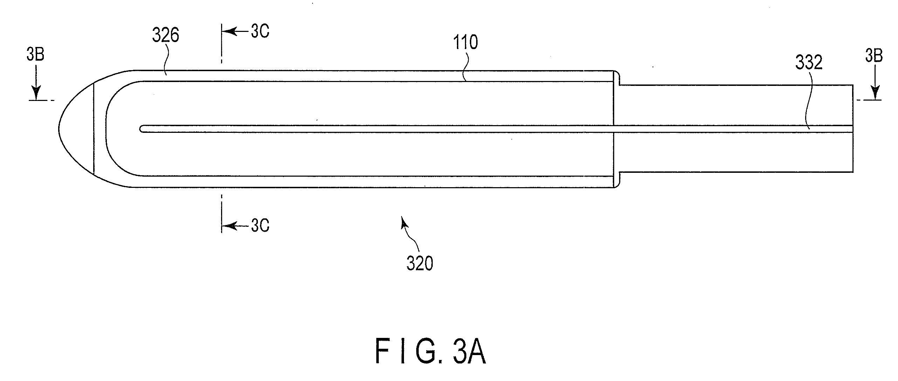

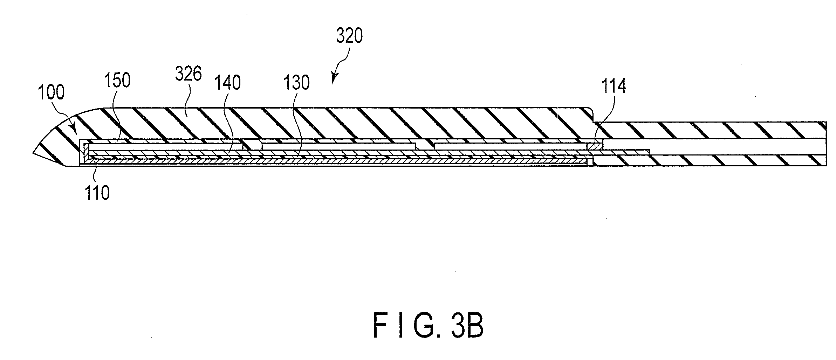

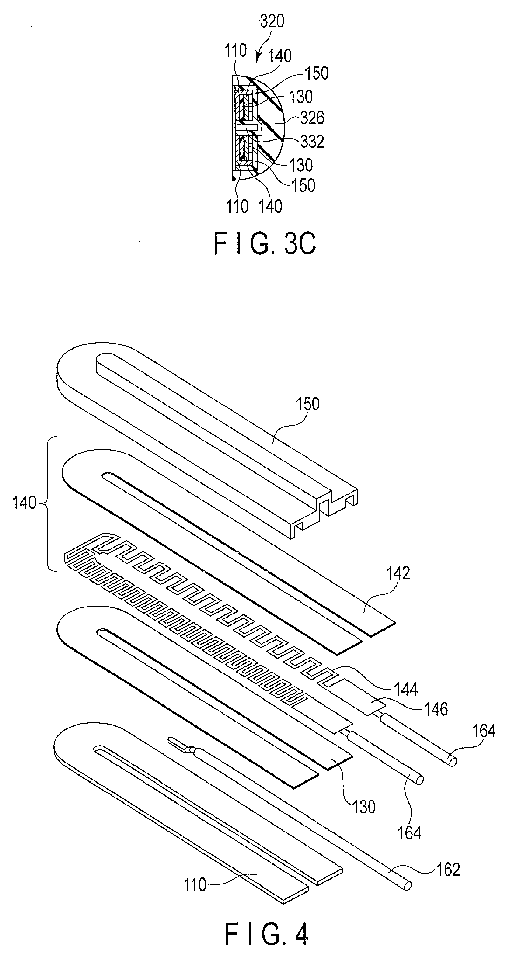

A structure of the first holding member 322 is schematically illustrated in FIG. 3A, FIG. 3B and FIG. 3C. FIG. 3A is a plan view of the first holding member 322 as viewed from a direction of the first high frequency electrode 110. FIG. 3B is a cross-section view along the line 3B-3B of FIG. 3A. FIG. 3C is a cross-section view along the line 3C-3C of FIG. 3A. As illustrated, the first holding member 322 is formed with the first cutter guide groove 332 for guiding the cutter 345.

The first holding member 322 is provided with the first high frequency electrode 110 including a conductive copper thin plate, for example. The first high frequency electrode 110 is configured to contact with a body tissue on either main surface thereof (which will be called first main surface below). The first high frequency electrode 110 includes the first cutter guide groove 332, and thus its planar shape is U-shaped as illustrated in FIG. 3A.

The first high frequency electrode 110 is electrically connected with the first high frequency electrode current line 162 to function as a lead wire for applying a high frequency voltage to the first high frequency electrode 110 in a way described later in detail. The first high frequency electrode 110 is connected to the control device 370 via the first high frequency electrode current line 162 and the cable 360.

The electrothermal conversion element 140 and a cover member 150 are arranged to a second main surface of the first high frequency electrode 110 which does not contact with a body tissue as described later in detail. A first electrode part 100 formed of the first high frequency electrode 110, the electrothermal conversion element 140, the cover member 150 and the like is formed in this way. The first electrode part 100 is embedded in and fixed on a first holding member main body 326. An exemplary structure of the first electrode part 100 will be described below in more detail.

As illustrated in FIG. 2A and FIG. 2B, the second holding member 324 is symmetrical in its shape to the first holding member 322, and has the same structure as the first holding member 322. That is, the second holding member 324 is formed with the second cutter guide groove 334 opposite to the first cutter guide groove 332. The second holding member 324 is provided with the second high frequency electrode 210 opposite to the first high frequency electrode 110. The second high frequency electrode 210 is configured to contact with a body tissue on either main surface thereof. The second high frequency electrode 210 is connected to the control device 370 via the second high frequency electrode current line 262 and the cable 360.

The electrothermal conversion element and a cover member are arranged to a surface of the second high frequency electrode 210 which does not contact with a body tissue. A second electrode part 200 formed of the second high frequency electrode 210, the electrothermal conversion element, the cover member and the like is formed in this way. The second electrode part 200 is embedded in and fixed on a second holding member main body 328.

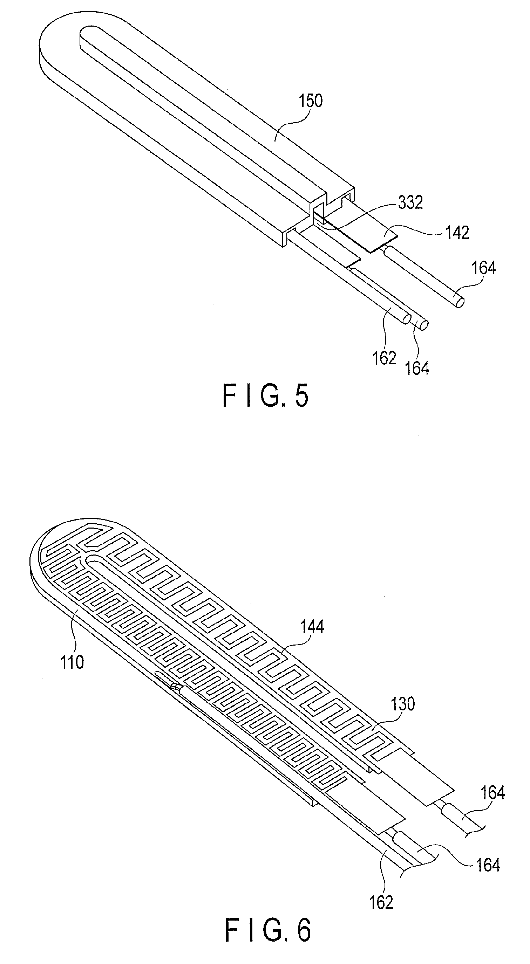

The first electrode part 100 will be described in detail. The second electrode part 200 has the same structure as the first electrode part 100, and thus the description of the second electrode part 200 will be omitted. An exploded perspective view of the first electrode part 100 is illustrated in FIG. 4. As illustrated, the first electrode part 100 includes the first high frequency electrode 110, a highly heat-conductive adhesive sheet 130, the electrothermal conversion element 140, and the cover member 150. The electrothermal conversion element 140 includes a substrate 142 and an electric resistance pattern 144. A perspective view of the first electrode part 100 assembled from the first high frequency electrode 110, the highly heat-conductive adhesive sheet 130, the electrothermal conversion element 140, and the cover member 150 is illustrated in FIG. 5.

As illustrated in FIG. 4, the first high frequency electrode 110, the highly heat-conductive adhesive sheet 130, and the electrothermal conversion element 140 have a U shape to form the first cutter guide groove 332. The cover member 150 has a shape including a groove to form the first cutter guide groove 332. As illustrated in FIG. 5, the first electrode part 100 forms the first cutter guide groove 332 as a whole. In addition, the first high frequency electrode current line 162 and first heater current lines 164 extend from a base end of the first electrode part 100.

As illustrated in FIG. 4, the electrothermal conversion element 140 has a substrate 142 including polyimide, for example. The shape of the substrate 142 generally matches with the shape of the first high frequency electrode 110 as illustrated in FIG. 4. The substrate 142 is slightly larger in length and slightly smaller in width than the first high frequency electrode 110. Parts of the electrothermal conversion element 140 that protrude from the first high frequency electrode 110 are referred to as extension parts.

An electric resistance pattern 144 is formed of a stainless (SUS) pattern, for example, in most of the substrate 142 except the extension parts. First lead connections 146 connected to both ends of the electric resistance pattern 144 are formed of a SUS pattern at the ends including the extension parts of the substrate 142. When a voltage is applied to a pair of first lead connections 146, the electric resistance pattern 144 generates heat. In this way, the electrothermal conversion element 140 functions as a sheet heater. A thickness of the electrothermal conversion element 140 is around 100 µm, for example.

The first high frequency electrode 110 and the electrothermal conversion element 140 are adhered to each other by the highly heat-conductive adhesive sheet 130. Herein, the electrothermal conversion element 140 is adhered with the surface forming the electric resistance pattern 144 thereon faced toward the first high frequency electrode 110. The highly heat-conductive adhesive sheet 130 is a sheet which is high in thermal conductivity and resistant to high temperature and has an adhesive property. The highly heat-conductive adhesive sheet 130 is made by mixing highly heat-conductive ceramic such as alumina or aluminum nitride with epoxy resin, for example. The highly heat-conductive adhesive sheet 130 has a high adhesive property, preferable thermal conductivity and an electric insulation property. A thickness of the highly heat-conductive adhesive sheet 130 is around 50 µm, for example.

The highly heat-conductive adhesive sheet 130 has substantially the same shape as the first high frequency electrode 110. The highly heat-conductive adhesive sheet 130 is slightly longer than the first high frequency electrode 110. Since the highly heat-conductive adhesive sheet 130 is longer than the first high frequency electrode 110, an electric insulation property between the first high frequency electrode 110 and the first lead connections 146 is secured.

The pair of first heater current lines 164 is connected to the pair of first lead connections 146. The first heater current lines 164 are connected to the surface with the electric resistance pattern 144 of the electrothermal conversion element 140 formed thereon, that is the surface opposed to the first high frequency electrode 110. A voltage is applied to the electric resistance pattern 144 from the first heater current lines 164 via the first lead connections 146 so that the electric resistance pattern 144 generates heat and the heat is transferred to the body tissue via the first high frequency electrode 110.

The electric resistance pattern 144 of the electrothermal conversion element 140 is arranged closer to the first high frequency electrode 110 than to the substrate 142 of the electrothermal conversion element 140, and arranged with the highly heat-conductive adhesive sheet 130 intervened between the electric resistance pattern 144 and the first high frequency electrode 110. Thus, the electric resistance pattern 144 is thermally coupled with the first high frequency electrode 110 via the highly heat-conductive adhesive sheet 130. Only the highly heat-conductive adhesive sheet 130 is present between the electric resistance pattern 144 and the first high frequency electrode 110, and thus heat generated by the electric resistance pattern 144 is efficiently transferred to the first high frequency electrode 110. The heat transferred to the first high frequency electrode 110 is transferred to a body tissue gripped by a holding part 320. As described above, the first high frequency electrode 110 also functions as a heat transfer plate to transfer the heat.

The first high frequency electrode current line 162 is further connected to the first high frequency electrode 110. The first high frequency electrode current line 162 is connected by soldering, for example, to a part of the second main surface of the first high frequency electrode 110, to which the highly heat-conductive adhesive sheet 130 and the electrothermal conversion element 140 are not adhered. A high frequency voltage is applied to the first high frequency electrode 110 from the first high frequency electrode current line 162 so that the first high frequency electrode 110 applies a high frequency current to a body tissue gripped by the holding part 320.

The cover member 150 is made of heat-resistant resin. The cover member 150 has a shape corresponding to the first high frequency electrode 110. A thickness of the cover member 150 is about 0.3 mm, for example.

In order to efficiently transfer heat generated by the electrothermal conversion element 140 to the first high frequency electrode 110, it is preferable that the cover member 150 and the first holding member main body 326 around the same have lower thermal conductivity than the first high frequency electrode 110 or the highly heat-conductive adhesive sheet 130. The cover member 150 and the first holding member main body 326 have low thermal conductivity so that loss of the heat generated by the electrothermal conversion element 140 is decreased.

The first high frequency electrode 110, the electric resistance pattern 144 of the electrothermal conversion element 140, and the first high frequency electrode current line 162 will be described in more detail. An arrangement of the electric resistance pattern 144 on the first high frequency electrode 110 and the first high frequency electrode current line 162 is illustrated in FIG. 6. While the highly heat-conductive adhesive sheet 130 is illustrated in the figure, for simplification, the substrate 142 of the electrothermal conversion element 140 is not illustrated.

As illustrated in FIG. 6, the first high frequency electrode current line 162 is arranged along the electrothermal conversion element 140 beside the electrothermal conversion element 140, parallel to a longitudinal direction of the first high frequency electrode 110. In addition, an electrical connection between the first high frequency electrode 110 and the first high frequency electrode current line 162 is arranged around the center with respect to the longitudinal direction of the first high frequency electrode 110. The electrical connection between the first high frequency electrode 110 and the first high frequency electrode current line 162 corresponds to a side of the region with the electric resistance pattern 144 formed in the electrothermal conversion element 140.

In addition, the electric resistance pattern 144 is formed in a corrugated shape. The line width of the electric resistance pattern 144 is uniform entirely. On the other hand, the electric resistance pattern 144 is formed such that the density thereof is higher on the side where the first high frequency electrode current line 162 is arranged with the first cutter guide groove 332 interposed therebetween, than on the side where the first high frequency electrode current line 162 is not arranged. More specifically, the pitch of the electric resistance pattern 144 formed around the first high frequency electrode current line 162 arranged is smaller than the pitch of the other part.

By forming the electric resistance pattern 144 as described above, the heat generation density of the electrothermal conversion element 140 increases in the part where the electric resistance pattern 144 is densely formed. More specifically, the heat generation density around which the first high frequency electrode current line 162 is arranged is higher than the heat generation density of the other part.

The first electrode part 100 has been described above, and the second electrode part 200 is the same as the first electrode part 100.

The operations of the treatment apparatus 300 according to the present exemplary embodiment will be described below. The operator previously operates the input part of the control device 370 to set the output conditions of the treatment apparatus 300, such as setting power for high frequency energy output, target temperature for thermal energy output, and heating time. The treatment apparatus 300 may be configured such that the respective values are independently set or a set of setting values is selected depending on an operation.

The holding part 320 and the shaft 340 in the energy treatment tool 310 are inserted into the abdominal cavity via the peritoneum, for example. The operator operates the operation knobs 352 to open/close the holding part 320 so that a body tissue to be treated is gripped by the first holding member 322 and the second holding member 324. At this time, the body tissue to be treated contacts on the first main surfaces of both of the first high frequency electrode 110 provided on the first holding member 322 and the second high frequency electrode 210 provided on the second holding member 324.

When the body tissue to be treated is gripped by the holding part 320, the operator operates the foot switch 380. When the foot switch 380 is tuned ON, high frequency power for preset power is supplied from the control device 370 to the first high frequency electrode 110 and the second high frequency electrode 210 via the first high frequency electrode current line 162 passing inside the cable 360 and the second high frequency electrode current line 262. The supplied power is approximately 20 W to 80 W, for example. Consequently, the body tissue generates heat and the tissue is cauterized. The tissue modifies and coagulates due to the cauterization.

After the control device 370 stops outputting high frequency energy, the electrothermal conversion element 140 is supplied with power such that the temperature of the first high frequency electrode 110 reaches a target temperature. Herein, the target temperature is 200°C, for example. At this time, a current flows through the electric resistance pattern 144 of the electrothermal conversion element 140 from the control device 370 via the cable 360 and the first heater current lines 164. The electric resistance pattern 144 generates heat due to the current. The heat generated by the electric resistance pattern 144 is transferred to the first high frequency electrode 110 via the highly heat-conductive adhesive sheet 130. Consequently, the temperature of the first high frequency electrode 110 increases.

Similarly, the electrothermal conversion element in the second electrode part 200 is supplied with power such that a temperature of the second high frequency electrode 210 reaches the target temperature. The electrothermal conversion element in the second electrode part 200 is supplied with power from the control device 370 via the cable 360 and the second heater current lines 264 so that the temperature of the second high frequency electrode 210 increases.

The body tissue contacting with the first high frequency electrode 110 or the second high frequency electrode 210 is further cauterized and further coagulated by the heat. When the body tissue coagulates by the heating, the control device 370 stops the output of thermal energy. The operator finally operates the operation knobs 352 to move the cutter 345, thereby cutting the body tissue. The treatment of the body tissue is completed with the above operations.

In the first electrode part 100 according to the exemplary embodiment, a high voltage is applied to the first high frequency electrode 110. For this reason, a relatively thick conducting wire is used for the first high frequency electrode current line 162. The first high frequency electrode current line 162 is a thick conducting wire, and thus excellent in thermal conductivity. Thus, the temperature of the first high frequency electrode 110 is likely to fall in the vicinity of the first high frequency electrode current line 162, due to the thermal conduction through the first high frequency electrode current line 162.

Therefore, according to the present exemplary embodiment, as explained with reference to FIG. 6, the density of the electric resistance pattern 144 formed around which the first high frequency electrode current line 162 is arranged is higher than that of the other part. The density of the electric resistance pattern 144 is higher than that of the other part, and the heat generation density of the electrothermal conversion element 140 in the vicinity of the first high frequency electrode current line 162 is thus higher than the heat generation density of the other part of the electrothermal conversion element 140. Consequently, the temperature of the first high frequency electrode 110 in the vicinity of the first high frequency electrode current line 162 is sufficiently increased in spite of release of heat by the first high frequency electrode current line 162. More specifically, the difference in temperature is small in the first high frequency electrode 110. The same applies to the second electrode part 200.

Further, according to the present exemplary embodiment, the electric resistance pattern 144 formed for the electrothermal conversion element 140 is formed such that the density of the side where the first high frequency electrode current line 162 is arranged with the first cutter guide groove 332 interposed therebetween is higher than the density of the side where the first high frequency electrode current line 162 is not arranged. However, without limitation thereto, for example, the electric resistance pattern 144 may be densely formed in the vicinity of the first high frequency electrode current line 162 on the side where the first high frequency electrode current line 162 is arranged from the first cutter guide groove 332, whereas the electric resistance pattern 144 may be sparsely formed at the part away from the first high frequency electrode current line 162. More specifically, the electric resistance pattern 144 may be formed such that the heat generation density is high in the vicinity of the first high frequency electrode current line 162, whereas the electric resistance pattern 144 may be formed such that the heat generation density is low at the part away from the first high frequency electrode current line 162.

In addition, heat is likely to be released at ends such as the tip end and base end of the first high frequency electrode 110. Thus, for compensating for the released heat, the electric resistance pattern 144 may be densely formed at an end of the electrothermal conversion element 140 to increase the heat generation density. As described above, the temperature of the first high frequency electrode 110 can become uniform, with the heat generation density increased by formation of the electric resistance pattern 144 more densely in locations where the heat of the first high frequency electrode 110 is likely to be released than at the other part. While an example of the fact that the heat generation density around the first high frequency electrode current line 162 arranged is higher than the heat generation density at the other part has been presented in the present exemplary embodiment, the present exemplary embodiment never exclude the inclusion of a part with a significantly higher heat generation density.

As described above, when the region where the electric resistance pattern 144 of the electrothermal conversion element 140 is formed regarded as a pattern region in the first electrode part 100 according to the present exemplary embodiment, the heat generation density associated with the electric resistance pattern 144 in a first region including a region where the distance from the first high frequency electrode current line 162 is smaller than a predetermined value is higher than the heat generation density associated with the electric resistance pattern 144 in a second region including at least a part of the pattern region other than the first region. In addition, the heat generation density associated with the electric resistance pattern 144 in a third region including a region where the distance from an end of the first high frequency electrode 110 is smaller than a predetermined value is higher than the heat generation associated with the electric resistance pattern 144 in the second region.

Further, the present exemplary embodiment has provided, but not limited to, the example where the first high frequency electrode current line 162 is connected to the first high frequency electrode 110 around the center of the first high frequency electrode 110 with respect to the longitudinal direction. The first high frequency electrode current line 162 just has to be provided in the vicinity of a region of the electric resistance pattern 144 formed in the electrothermal conversion element 140, and the electric resistance pattern 144 in the vicinity of the first high frequency electrode current line 162 just has to be densely formed that electric resistance pattern 144 in at least a region of the other part.

According to the present exemplary embodiment, as illustrated in FIG. 6 and the like, the pitch of the electric resistance pattern 144 formed in a corrugated shape is varied to cause the heat generation density to differ depending on sites of the electrothermal conversion element 140, thereby making the temperature of the first high frequency electrode 110 uniform. However, the method for varying the heat generation density is not limited thereto. For example, for a region where the heat generation density is desired to be increased in the electrothermal conversion element 140, the line width of the electric resistance pattern 144 may be reduced to increase the electric resistance, and thus increase the amount of heat generation of the electrothermal conversion element 140. Alternatively, the amount of heat generation may be adjusted by adjusting both the pitch and line width of the electric resistance pattern 144.

### Second Exemplary Embodiment

A second exemplary embodiment will be described. Herein, the differences from the first exemplary embodiment will be described, and the same parts are denoted with the same reference numerals and the description thereof will be omitted. In the present exemplary embodiment, a first electrode part 100 differs from the first electrode part 100 according the first exemplary embodiment in the structure of first high frequency electrode 110, electrothermal conversion element 140, and first high frequency electrode current line 162.

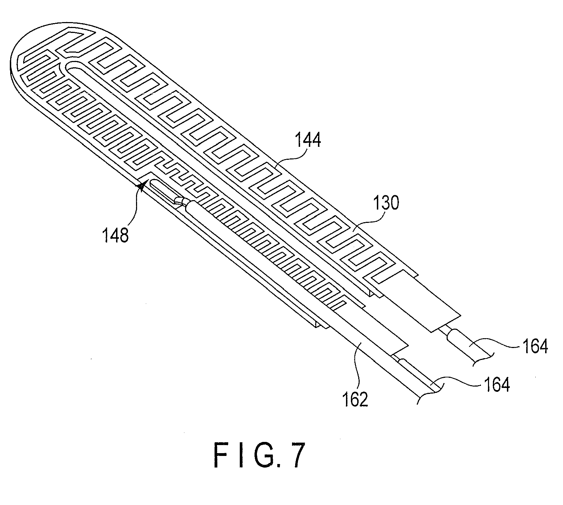

The structures of the first high frequency electrode 110, electrothermal conversion element 140, first high frequency electrode current line 162, and the like in the first electrode part 100 according to the present embodiment are illustrated in FIG. 7. As illustrated in FIG. 7, the electrothermal conversion element 140 and highly heat-conductive adhesive sheet 130 according to the present exemplary embodiment are provided with a cutout part 148. The first high frequency electrode current line 162 is connected to the first high frequency electrode 110 at the cutout part 148. In addition, the electric resistance pattern 144 of the electrothermal conversion element 140 according to the present exemplary embodiment is densely formed in the vicinity of the cutout part 148 more than at the other part.

In accordance with the present exemplary embodiment, the first high frequency electrode current line 162 is connected to the first high frequency electrode 110 at the cutout part 148, and thus, at the part, heat is likely to be released from the first high frequency electrode current line 162. In addition, at the cutout part 148, because of the reduced region where the electric resistance pattern 144 is formed, the amount of heat generation is likely to be relatively lowered. Therefore, according to the present exemplary embodiment, the electric resistance pattern 144 is densely formed in the region with the cutout part 148. More specifically, the density of heat generation by the electric resistance pattern 144 is higher in the region with the cutout part 148 than in other region.

The first high frequency electrode current line 162 can be arranged to have an overlap with the electrothermal conversion element 140, by providing the electrothermal conversion element 140 with the cutout part 148 as in the present exemplary embodiment. Consequently, the first electrode part 100 can be reduced in size. In addition, the heat generation density of the electrothermal conversion element 140 is adjusted to compensate for a decrease in heat generation density, which is caused by providing the electrothermal conversion element 140 with the cutout part 148, and release of heat, which is caused by the presence of the first high frequency electrode current line 162. Therefore, the first high frequency electrode 110 is less likely to produce a difference in temperature.

An example of providing a cutout part at an edge of the electrothermal conversion element 140 has been given as illustrated in FIG. 7 in the present exemplary embodiment. However, without limitation thereto, the first high frequency electrode current line 162 may be connected to the first high frequency electrode 110 at an opening provided in the electrothermal conversion element 140 in place of the cutout part.

### Reference Signs List

- 100: first electrode part
- 110: first high frequency electrode
- 130: highly heat-conductive adhesive sheet
- 140: electrothermal conversion element
- 142: substrate
- 144: electric resistance pattern
- 146: lead connection
- 148: cutout part
- 150: cover member
- 162: first high frequency electrode current line
- 164: first heater current line
- 200: second electrode part
- 210: second high frequency electrode
- 262: second high frequency electrode current line
- 264: second heater current line
- 300: treatment apparatus
- 310: energy treatment tool
- 320: holding part
- 322: first holding member
- 324: second holding member
- 326: first holding member main body
- 328: second holding member main body
- 332: first cutter guide groove
- 334: second cutter guide groove
- 340: shaft
- 342: tube
- 343: sheath
- 344: drive rod
- 345: cutter
- 346: support pin
- 347: elastic member
- 350: handle
- 352: operation knob
- 360: cable
- 365: connector
- 370: control device
- 380: foot switch

## Claims

1. A treatment apparatus configured to treat a body tissue, the treatment apparatus comprising:
a conductive heat transfer plate configured to transfer heat and power to the body tissue in contact with the body tissue;
an electrothermal conversion element provided on the heat transfer plate, the electrothermal conversion element comprising an electric resistance pattern that generates heat with a voltage applied; and
a lead wire configured to supply an electric current to the heat transfer plate, wherein
heat generation density by the electric resistance pattern in a first region of a pattern region where the electric resistance pattern of the electrothermal conversion element is formed, the first region comprising a region where a distance from the lead wire is smaller than a predetermined value, is higher than heat generation density by the electric resistance pattern in a second region comprising at least a part of the pattern region other than the first region.

2. The treatment apparatus according to claim 1, wherein the electric resistance pattern in the first region has a smaller pitch than the electric resistance pattern in the second region, thereby the heat generation density of the first region thus is higher than the heat generation density of the second region.

3. The treatment apparatus according to claim 1, wherein the electric resistance pattern in the first region has a smaller line width than the electric resistance pattern in the second region, thereby the heat generation density of the first region is higher than the heat generation density of the second region.

4. The treatment apparatus according to any one of claims 1 to 3, wherein
the electrothermal conversion element is provided with a cutout part or an opening, and
the lead wire is connected to the heat transfer plate at the cutout part or the opening.

5. The treatment apparatus according to any one of claims 1 to 3, wherein heat generation density by the electric resistance pattern in a third region of the pattern region, the third region comprising a region where a distance from an end of the heat transfer plate is smaller than a predetermined value is higher than heat generation density by the electric resistance pattern in the second region.
